# EUROPEAN PATENT APPLICATION

(11) **EP 1 482 309 A1**
(43) Date of publication of application: **01.12.2004**
(21) Application number: 03291096.0
(22) Date of filing: 07.05.2003
(51) Int. Cl.: G01N 33/53, C07K 16/18, G01N 33/68

(54) **Means for detecting protein conformation and applications thereof**

(71) Applicant: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: Perez, Franck, 75013 Paris (FR); Goud, Bruno, 75015 Paris (FR)
(74) Representative: Peaucelle, Chantal

(57) **Abstract**

The invention relates to the use of scFv fragments as conformation-specific antibodies for specifically detecting a conformational protein state.

Application as sensors for following in living cells, upon intracellular expression, the behaviour of endogeneous proteins.

## Description

The invention relates to a method comprises the use of recombinant antibodies as sensors for specifically detecting a particular protein conformation and their applications in normal and pathological applications.

Most of the proteins oscillate between inactive and active conformation to fulfill their activity. The specific detection of a particular protein conformation is then a key step in understanding protein functions in various conditions.The conformational change generally triggers the function of downstream effectors. To analyze the spatio-temporal activation of proteins, binding domains of effector proteins have been used as conformation sensors with the limitation that these effector-based conformation sensors necessarily compete with endogenous effectors for protein binding, and in turn affect the protein function itself. This approach was shown to be very powerful to study the activation of small GTPase.

The inventors decided to develop a method to design protein conformation sensors that would not require the knowledge of an existing effector, and that would not necessarily lead to inhibition of effector binding to the activated protein and used the antibody phage display.

Antibodies have been extensively used to identify protein both qualitatively with respect to their nature and localization, and quantitatively.

More recently, *a*lternative *in vitro* approach have been developped to select for recombinant antibody fragment.

The antibody phage display was shown to be both a fast and powerful approach to select for Fab or Fv fragments of immunoglobulins that are presented at the surface of filamentous phages.

The inventors have now shown that recombinant antibodies of great interest could be obtained by such a method and were able to detect a given conformational protein state even when said protein was naturally expressed.

Accordingly, an object of the invention is to provide means, particularly tools and methods, for specifically detecting a given protein conformation in various conditions, *in vitro* and *in vivo* by using such recombinant antibodies.

Another object of the invention is to provide a method using said recombinant antibodies as sensors for detecting the localization of the proteins and follow in living cells the behaviour of endogeneous proteins.

The invention thus relates to the use of the scFv fragments as conformation-specific antibodies for specifically detecting a conformational protein state.

Advantageously, said scFv are selected from a combinatorial library of scFv presented at the surface of phages.

Such a selection allows the extensive control of *in vitro* selection conditions. This has the major advantage, in comparison to classical - animal-based - approaches, of preserving the native conformation of target antigens. In addition, scFvs have been used widely as intrabodies to target intracellular proteins (9, 10), even though scFvs are often inactive in vivo because of misfolding induced by the reducing environment of the cytosol (*11*).

The method of the invention comprises the use of said scFv in immunofluorescence to determine the subcellular localization of the protein in a specific conformation.

Such means is an essential step for the understanding of protein function in normal and pathological conditions.

The invention also relates to the use of said scFv fragments for detecting the expression of mutated proteins locked in a particular conformation.

The invention thus provides means for detecting the presence of mutated proteins in certain pathologies where no overexpression could be detected when looking at the mRNA level. For example, dominant active Ras, found in many cancers, may be detectable, while no difference would be visible by using a cDNA chip.

Acccording to another embodiment, the invention also relates to the use of said scFv fragments for detecting by immunoprecipitation the presence of proteins in particular conformation.

It is then possible to detect the presence of mutated proteins in conditions where no overexpression at the nucleic acid level would be visible. The inventions thus provides means for setting up diagnostic protocols based on the dosage of particularly conformed proteins.

The invention also relates to the use of said scFv as sensors for following in living cells, upon intracellular expression, the behavior of endogeneous proteins.

The dynamics of proteins is usually followed *in vivo* upon overexpression of that protein in fusion with fluorescent markers. The invention alows to follow endogeneous protein dynamics, hence avoiding the potential artefact due to the overexpresion of proteins tagged with fluorescent markers.

Other characteristics and advantages of the invention will be disclosed hereinafter, with reference to a recombinant antibody against the Golgi-localized small GTPase Rab6A that regulates the traffic between the Golgi apparatus and the endoplasmic reticulum.

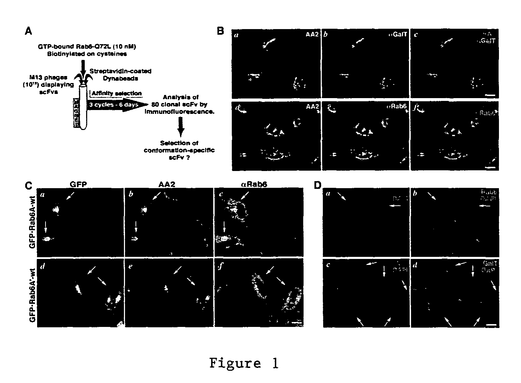

In said experiments, it will be referred to figures 1 to 4 and movies S 1 and S2. Said figures and movies are going to be published in Science, in the issue of May 2003. They respectively represent
**Fig. 1:** *in vitro* selection of anti-Rab6•GTP recombinant antibodies by phage display.
   **A**. A combinatorial recombinant antibody library was screened against Rab6AQ72L (GTP-bound)
   **B**. AA2 staining colocalizes with Rab6 on the Golgi. HeLa cells were stained with the recombinant antibody AA2, polyclonal anti-GalT (a-c) or anti-Rab6 (d-f) antibodies, and DAPI (nuclei staining, blue in the overlay (c, f)). Arrows indicate peripheral structures positive for both AA2 and the anti-Rab6.
   **C**. AA2 detects Rab6A and A'. HeLa cells were transfected with GFP-Rab6A (a-c) or GFP-Rab6A' (d-f) and co-stained with AA2 and an anti-Rab6 polyclonal. Transfected cells are shown by arrows.
   **D**. AA2 staining is sensitive to Rab6A/A' knock-down. HeLa cells were treated or not with siRNA for 72 hr (arrows) to deplete both Rab6 isoforms and stained with AA2 (a, c) together with an anti-Rab6 (b) or an anti-GalT (d) antibody. Bars =10 µm.

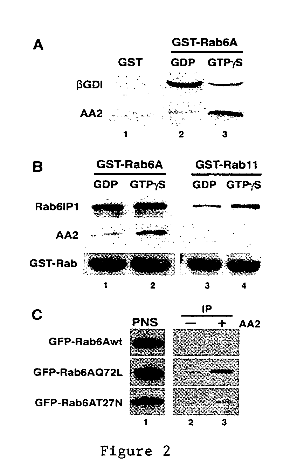

**Fig.2:** AA2 specifically interacts with Rab6•GTP in vitro.
   **A**. Recombinant Rab6A-GST coupled to Glutathion-sepharose was loaded with GDP (lane 2) or GTPγS (lane 3), and incubated with AA2 in the presence of trace amounts of mouse brain PNS. As a control, GST alone was incubated with AA2 (lane 1). Protein complexes were recovered by centrifugation and analyzed by western blotting. βGDI, present in the mouse brain PNS, binds preferentially to Rab6A•GDP while AA2 (detected with the 9E10 anti-myc tag antibody) binds preferentially to Rab6A•GTPγS.
   **B.** The same pull-down experiment was done using GST-Rab6A (lane 1,2) or GST-Rab11 (lane 3,4) in the absence of PNS, but using Rab6IP1 as a control effector protein. While Rab6IP1 binds to both Rab6A and Rab11, AA2 only binds to Rab6A•GTPγS.
   **C.** Immunoprecipitation experiments were carried out without (lane 2) or with AA2 (lane 3) from detergent extracted PNS (lane 1) of HeLa cells transiently expressing GFP-Rab6wt, GFP-Rab6T27N or GFP-Rab6Q72L. AA2 specifically precipitates GFP-Rab6Q72L and only traces of GFP-Rab6wt or GFP-Rab6T27N.

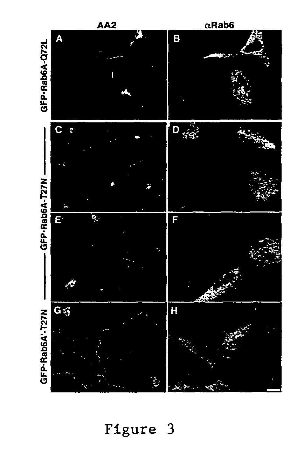

**Fig.3:** AA2 specifically detects Rab6•GTP by immunofluorescence. HeLa cells expressing GFP-tagged Rab6AQ72L (A, B) or Rab6A/A'T27N (C-H), were co-stained with AA2 and an anti-Rab6 polyclonal. Transfected cells, identified using the GFP staining, are outlined (A, C, E, G). While the anti-Rab6 polyclonal detects all forms of Rab6 expressed, AA2 detects GFP-Rab6AQ72L (A, B) on the Golgi only and not GFP-Rab6A/A'T27N (C-H). Endogenous Rab6 staining of the Golgi is only partially displaced upon Rab6AT27N over-expression (F). In contrast, total displacement was observed in certain cells expressing Rab6A'T27N (G). Bar=10 µm.

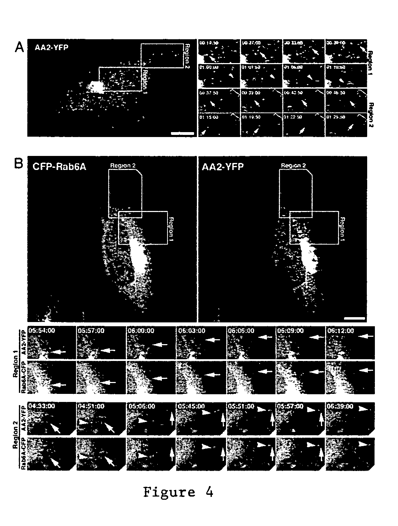

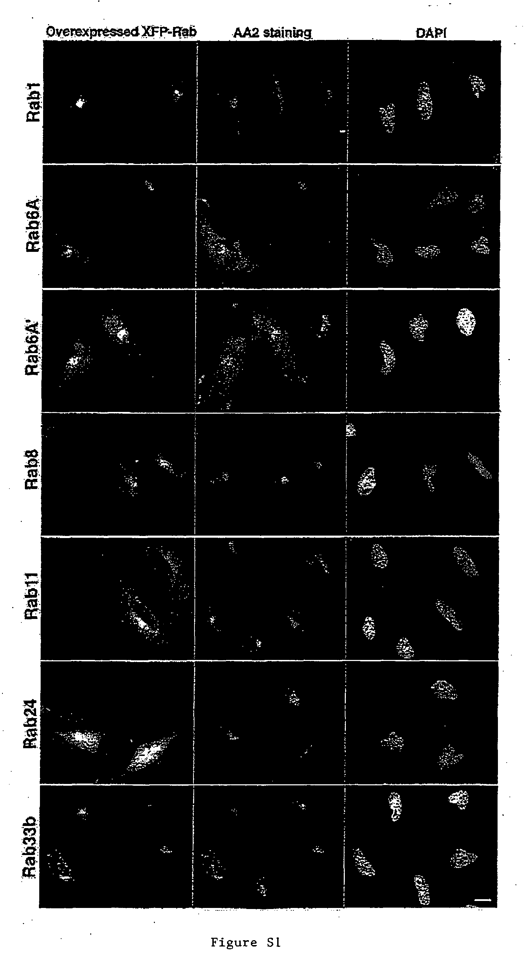

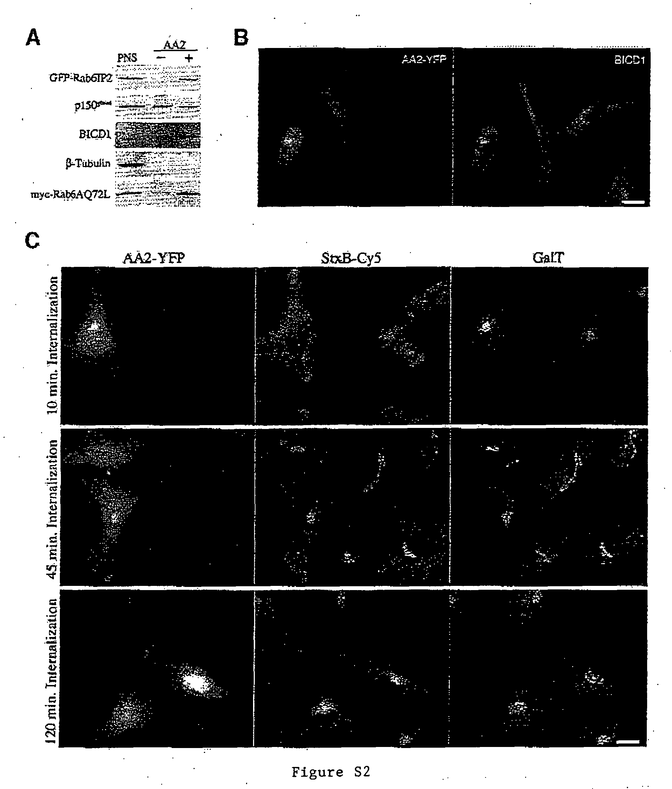

**Fig.4:** Endogenous and overexpressed Rab6•GTP dynamics in vivo. HeLa cells expressing either AA2-YFP alone (A) or AA2-YFP and CFP-Rab6 **(B)** were time-lapse imaged on a confocal microscope. The boxed regions in (A) and (B) are shown in a time-lapse series underneath. Region 2 in **(B)** was rotated by 90° clockwise.
   **A.** AA2-YFP detects endogenous Rab6•GTP in living cells on the Golgi and 1-2 µm-long tubulo-vesicular structures moving rapidly in the cytoplasm. In region 1, structures emerge from the Golgi and move towards either the cell periphery (arrow) or the cell center (arrowhead). Region 2 shows dynamics in a peripheral region likely to be an ER entry site, containing rather immobile structures (bracket) and towards which many structures converge (arrows).
   **B**. CFP-Rab6 is present on the Golgi and the ER, and on 5-20 µm-long membrane tubules (arrows) moving from the Golgi towards the periphery, with some returning. AA2-YFP staining shows that Rab6 is present in its GTP-bound form on the Golgi and all motile structures. Bar=10 µm.

Fig. S1: AA2 only detects overexpressed Rab6A and A' and not various other Rabs. HeLa cells were transfected with expression plasmids encoding GFP or YFP-fusions of various Rabs (left column, the transfected Rab is indicated on the left), fixed with 3% paraformaldehyde and stained with AA2 (middle column) and DAPI to stain nuclei (right column). AA2 detects overexpressed Rab6A and Rab6A', but not the other Rabs tested, including those that are at least partially localized to the Golgi.

**Fig. S2:** AA2 does not interfere with Rab6 function in intracellular traffic. A. AA2 co-precipitates Rab6•GTP and at least three of its effectors. Rab6•GTP was immunoprecipitated with AA2 from detergent extracts of HeLa cells expressing myc-tagged Rab6AQ72L (and Rab6IP2-GFP). Bound and unbound fractions were analyzed by western blotting (as well as an aliquot of the PNS as positive control). Rab6, Rab6IP2-GFP, endogenous p150^{glued} and endogenous BICD1 (but not endogenous βtubulin, negative control) are co-precipitated. Note that BICD 1 is even strongly enriched (not detected in the PNS aliquot) upon Rab6•GTP precipitation. Altogether, this suggests that AA2 binding to Rab6•GTP does not compete with that of Rab6 effectors. B. The Golgi localization of BICD1, an effector of Rab6, is not affected by AA2-YFP expression. HeLa cells expressing AA2-YFP were fixed with 3% paraformaldehyde in PBS and stained for BICD1. In contrast, a dominant negative mutant of Rab6, Rab6AT27N, was shown to affect Rab6 function and displace its effector BICD1 from the Golgi *(S8).* C. The retrograde transport of Shiga toxin B subunit (SStxB, 9) from the plasma membrane to the ER via the Golgi is not affected by AA2-YFP expression. It has been shown that Rab6A and A' are involved in the retrograde transport of the StxB from the plasma membrane to the Golgi and the ER: Rab6AT27N and Rab6A'T27N dominant negative mutants affect Rab6 function and prevent the transport of StxB from the Golgi to the ER and from the endosomes to the Golgi respectively *(S9,* 10). We used this assay to follow Rab6 activity in AA2-YFP-expressing cells. HeLa cells expressing AA2-YFP were continuously incubated with 5 µg/mL Cy5-coupled Shiga toxin B subunit, fixed with 3% paraformaldehyde in PBS after 10 min, 45 min or 120 min and stained for GalT. StxB is normally transported from the plasma membrane to endosomes (10 min), the Golgi (reached at 30-45 min, and continuously refilled from the surface thereafter) and then the ER (barely visible at 45 min, clearly visible at 120 min at the nuclear envelope) in all cells, regardless of AA2-YFP expression. Results in B and C indicate that AA2-YFP expression does not interfere with Rab6 A and A' functions in intracellular transport.

### Movie S1 (and movie S3 as a less compressed version): Corresponding to Figure 4A

Confocal imaging of a live HeLa cell expressing AA2-YFP (1 frame every 1.5 s, movie played twice).

AA2-YFP labels Rab6•GTP on the Golgi, as well as vesicles and short 1-2 µm-long tubules (arrows) moving towards the periphery at putative ER entry points (brackets) containing rather immobile Rab6•GTP-positive structures. Some tubulo-vesicular structures move back towards the Golgi (arrows).

### Movie S2 (and movie S4 as a less compressed version):Corresponding to Figure 4B

Confocal imaging of live HeLa cells expressing CFP-Rab6A and AA2-YFP (1 frame every 3 s, movie played twice).

CFP-Rab6A labels the Golgi and the endoplasmic reticulum (nuclear envelope), and 5-20 µmlong tubules (arrows) moving towards the periphery at putative ER entry points, and sometimes back to the Golgi. CFP-Rab6A dynamics match those already reported for GFP-Rab6A *(S11).* AA2-YFP labels Rab6 in its GTP-bound form on the Golgi and also on all tubules all along their length. Note however that no signal is detected on the ER.

In vitro selection of anti-R by phage display
- a combinatorial recombinant library was screened against R mutant(GTP bound)
- Ab staining

### A - Materials

The following materials were used in the experiments disclosed hereinafter:

### Antibodies.

Anti-βGDI (*S1*), anti-Rab6IP1 and anti-Rab6 were raised in rabbits.

Anti-GalT was kindly provided by E.G. Berger (University of Zurich, Zürich, CH), but Golgi apparatus can be stained by using other available, appropriate antibodies; anti-BICD1 was provided by C. Hoogenraad (Erasmus University, Rotterdam, NL). The anti-GFP antibody was from ROCHE, the anti-βtubulin from SIGMA, the anti-p150^{glued} from BD-Transduction Laboratories. Fluorescent secondary antibodies were from Jackson(?) (Cy3 and Cy5-labelled) and

Molecular Probes (Alexa488). HRP-coupled secondary antibodies were from Jackson Immunochemicals. Purified Shiga toxin was obtained from L. Johannes (Institut Curie, Paris, F).

### . Vectors

Vectors used to express fluorescent Rab6A and A' wt and mutant proteins were obtained from J.

White (EMBL, Heidelberg, D), A. Couedel-Courteille, A. Echard and F. Jollivet (Institut Curie, Paris, F). Vectors used to express fluorescent Rab1, Rab8, Rab11, Rab24, and Rab33b were respectively obtained from R. Sannerud & J.Saraste (University of Bergen, Norway), J. White (EMBL, Heidelberg, D), F. Senic-Matuglia & J. Salamero (Institut Curie, Paris, F), D. Munafo & M. Colombo (Universidad Nacional de Cuyo, CONICET, Mendoza, Argentina) and J. Young & T. Nilsson (EMBL, Heidelberg, D).

### . Library

G. Winter at the MRC (Cambridge, UK) provided us with the Griffin. 1 library as well as with TG1 and HB2151 E. coli strains.

### B - Methods

### In vitro binding.

Rab6-GST pull-down experiments were carried out as described (*S1*). Briefly, Rab6-GST purified from E. *coli* was coupled to Gluthation-sepharose beads (12.5 µg per condition), loaded with GDP or GTPγS in 10 mM EDTA buffer for 1hr at 37°C and then incubated with 0.2 µg AA2 and either 10 µg mouse post nuclear supernatant (PNS), 1.5 µL cytosol of Rab6IP1-expressing SF9 insect cells, 0.1% casein, 1% BSA or no blocking agent for 90 min at room temperature in the presence of 10 mM MgCl₂. Beads were washed 5 times and processed for SDS-PAGE. Bound and unbound fractions were analyzed by western blotting. The AA2-Rab6•GTPγS interaction was identical in all 5 conditions tested. βGDI, Rab6IP1 and AA2 were detected with an anti-GDI antibody (*S1*), an anti-Rab6IP1 antibody and the 9E10 monoclonal anti-myc-tag antibody respectively, followed by HRP-labelled secondary antibodies and chemiluminescence staining (Pierce).

**Cell culture and Immunoprecipitation.** HeLa cells were cultured as described in Mallard et al. *(S5)* and transfected using CaPO₄ *(S6).* For immunoprecipitation experiments, 15.10⁶ HeLa cells were transfected, and after 16 hr, were detached from culture flasks in PBS-EDTA, pelleted and resuspended in 3 mM imidazol pH=7.2, 30 mM MgCl₂ and proteases inhibitors. Cells were lysed with a ball-bearing cell cracker on ice, adjusted to 150 mM NaCl, 20 mM Imidazol and 0.5% Triton X100, centrifuged at 1000 g for 5 min and the resultant PNS was incubated with or without 50 µg AA2 at 4°C for 4 hr. 150 µL of washed Ni-NTA magnetic beads (Qiagen) were then added to reactions for 1 hr at 4°C, washed 5 times in PBS, 20 mM Imidazol, 30 mM MgCl₂, 0.5% Triton X100, and bound and unbound fractions were processed for SDS-PAGE using anti-BICD1 #2296, anti-GFP, anti-βtubulin and anti-p150^{glued}.

**siRNA** . Rab6 knock down experiments were performed in HeLa cells essentially as described by Elbashir et al. *(S7)* using double stranded RNA oligos (Dharmacon) and will be described elsewhere. Rab6 was depleted in nearly 100% of cells incubated with the siRNA duplex for three days. siRNA-treated and wt, non-treated , HeLa cells were thus mixed on the same cover slips 24 hr after the siRNA transfection,. This ensured that control, non-Rab6-depleted cells, would be visualized simultaneously as Rab6-depleted cells (as shown in Figure 1D). Cells were fixed with 3% paraformaldehyde 48 hr later (i.e. 72 hr siRNA-depletion) and processed for immunofluorescence. siRNA depletion of Rab6A/A' abolished AA2 Golgi labeling while specific siRNA-depletion of either Rab6 A or A' only reduced, but did not abolish, AA2 staining.

### Intracellular expression of AA2.

The NotI site downstream of thJe fluorescent protein cDNA in pECFP-N3, pEGFP-N3, pEYFP-N3 (BD-Clontech) were mutated by filling-in. The mutated vectors were then modified by inserting a synthetic adaptor containing a His₆-tag and a myc-tag, downstream of a NotI site, identical to the tags found in pHEN2. To allow for insertion of the scFv extracted from pHEN2 by NcoI/NotI, the synthetic adaptor also inserted an upstream BbsI/NcoI site so that cutting the modified vector with BbsI opens the vector inside the NcoI site exactly as if using NcoI itself. The fact that we conserved the myc- and His₆-tags and in general the entire context of AA2 as it was in the original pHEN2 vector (Griffin.1 library) may explain why AA2 (and other scFvs selected in our laboratory) works as an intrabody without any further modification in spite of the reducing chemical environment of the cytosol. It is however worth mentioning, while many of our scFvs detect their target proteins in vivo, none of them so far interferes with the function of endogenous proteins.

Live cell imaging.

HeLa cells cultured on cover slips were transfected using CaPO₄. After 16 hr, cover slips were mounted on an incubating chamber filled with culture medium supplemented with 20 mM Hepes pH=7.2 at 37°C. Time-lapse images were acquired on a Leica SP2 confocal microscope every 1.5 s or 3 s for 1 or 2 channel-recordings respectively. Both channels were acquired simultaneously using the 457 nm and 514 nm laser wavelengths for CFP and YFP respectively. Excitation intensities and spectral detection windows were systematically independently adjusted (using the AOTF and the spectral set-up respectively) so as to optimize signals and avoid crosstalk between channels. The absence of crosstalk was confirmed by imaging cells expressing either CFP-Rab6 or AA2-YFP only. 12-bit images were processed into Quicktime movies or still image samples using NIH Image J available at http://rsb.info.nih.gov/ij/ and Adobe Image Ready. CFP-Rab6 fluorescence signals were median-filtered (radius=1 pixel) to increase signal to noise ratio.

### C - Use of Rab6Q72L as a target antigen to select for recombinant scFvs

### a) Rab6Q72L purification and biotinylation

Rab6AQ72L (a GTP-locked mutant of Rab6A) cloned in pET15b vector was expressed and purified by Ni-NTA (Qiagen) affinity chromatography essentially as described for Sar1 and Rab1 (S2, 3). The cystein-specific coupling agent PEO-Maleimide activated biotin (PEO-biotin, Pierce) was used to biotinylate Rab6AQ72L.. Bacterially expressed Rab6AQ72L was modified in PBS supplemented with 0.5 % Tween20 in the presence of a 50x molar excess of PEO-biotin overnight at 4°C. 50% of the proteins were modified as estimated by recovery on streptavidin-coated beads and western blot analysis of bound and unbound fractions. Finally, biotinylated Rab6AQ72L was separated from free biotin using a Sephadex G25 column.

### b) Antibody Phage display screen.

The protocols provided with the Griffin.1 library and available on G. Winter's laboratory web page at http://www.mrc-cpe.cam.ac.uk/winter-hp.php?menu=1808 were followed.

The screen consisted of three rounds of selection, each of which took 2 days and used 250 ng biotinylated Rab6 (hence 10 nM of biotinylated Rab6 during the selection). The selection process was modified as follows. Phages (10¹⁴) were first incubated for 90 min with 50 µL washed streptavidin M280 dynabeads (DYNAL), beads were separated on a magnet and the non-pre-adsorbed phages were recovered. They were then slowly agitated for 30 min in the presence of 10 nM biotinylated Rab6AQ72L (in 1 mL PBS, 0.1% Tween20, 2% Marvel fat free milk), and the interaction was then left standing for 90 min. 50 µL washed streptavidin-coated dynabeads were then added, tubes were slowly agitated for 30 min and incubation was continued for 30 min standing. Beads were recovered and washed with PBS+Tween 10 times on a magnet. Phages were eluted with 100 mM Tri-ethylamine at pH=11 (twice with 500 µl for 10 min) and neutralized in 500 µl 1M Tris pH=7.4.

After the third round of selection, when the selection yield increase indicated positive selection, 80 clones were randomly picked from E. *coli* populations infected by the selected phages. These clones were analyzed by immunofluorescence for Golgi staining, and 37 clones were positive, labeling the perinuclear region of cells.

DNA sequencing of the 19 clones giving the strongest Golgi staining demonstrated that they were either one of two antibodies, AA2 or AH12. Similar results were obtained for AA2 and AH12, and only results concerning AA2 are shown here. The same antibodies were actually obtained when using 50 nM of target proteins instead of 10 nM in another independent screen. Accession number for AA2 in Genbank is AY265355.

### scFv production and immunofluorescence.

scFvs from the Griffin. 1 library are cloned in pHEN2 which allows the production of scFv-M13 pIII fusion proteins in amber suppressor E. *coli* strains (e.g. SupE) and the production of soluble scFvs in non-suppressor strains. Selected plasmids were thus introduced in the non-suppressor HB2151 strain before production.

For the immunofluorescence screen, scFvs secreted in 1 mL 2xTY culture (produced in 96-deep well plates), after overnight induction with 1 mM IPTG at 30°C, were used undiluted without purification. For large scale production, clones were grown at 37°C in 500 mL 2xTY medium supplemented with Ampicillin and 1% Glucose, spun at OD=0.2, resuspended in minimal M9 medium supplemented with Ampicillin, 1 mM IPTG, 0.5 ppm thiamin, 0.1% casa-aminoacids, and 0.2% glycerol as carbon source, and incubated for 16 hr at 30°C. Bacteria were pelleted and the supernatant filtered (Millipore 0.2 µm). scFvs secreted in the culture medium were concentrated on 2 mL 50% slurry Ni-NTA agarose columns (Qiagen).

For immunofluorescence of paraformaldehyde-fixed cells permeabilized with saponin (0.05%), purified scFvs were used at 10 µg/mL (or undiluted when culture medium was directly used) co-incubated with 9E10 anti-myc and/or anti-His₆ (SIGMA) monoclonals in the presence of 0.2% BSA, followed by anti-mouse fluorescent secondary antibodies.

Cells were imaged on a Leica DMRA microscope equipped with fluorescence filters (Chroma, Leica) and a CCD camera (MicroMax, Princeton). Images were acquired using Metamorph (Universal Imaging) and figures were assembled using Photoshop 7.0 (Adobe) on a G4 Macintosh (Apple).

The results obtained by immunofluorescence are given on Figure 1B a-c.

AA2 stains the Golgi complex positive for the Galactosyl Transferase (GalT). Extensive colocalization was observed when co-staining with AA2 and an anti-Rab6 polyclonal (Figure 1B d-f), including at peripheral sites, likely to be ER entry points (Figure 1B d-f, arrows).

Two spliced variants of Rab6 are expressed in cells, Rab6A and Rab6A' (*14*). AA2 detected both transfected Rab6A/A' (Figure 1C, on the Golgi but not the ER), but not other overexpressed Rabs (Figure S1). AA2 Golgi staining was completely abolished upon simultaneous Rab6A and A' siRNA-depletion (Figure 1C a, b; arrows) whereas that of GalT was not altered (Figure 1D c, d). Thus AA2 was directed against Rab6A and Rab6A'.

### d) specific detection of Rab6 in its GTP-bound conformation (Figure 2).

GST-Rab6A bound to glutathion-Sepharose® was loaded with GDP or with GTPγS, incubated with AA2 and recovered by pull-down. The protein βGDI (***15***), present in the post-nuclear supernatant (PNS) used as a control, bound more strongly to the GDP-bound form of Rab6A (***16***). In contrast, AA2 interacted preferentially with the activated GTPγS-bound form of Rab6 (Figure 2A). GTPγS-loaded Rab11 did not bind to AA2, although Rab6IP1, which is an effector of both Rab6 and Rab11 (Figure 2B), bound to both Rab6A and Rab11 in these conditions. Thus AA2 is an anti-Rab6 antibody directed against the GTP-bound conformation.

### e) immunoprecipitation experiments

AA2 binding to Rab6•GTP was also observed in immunoprecipitation experiments using transfected HeLa cells extracts. The results are illustrated by Figure 2C. GFP-Rab6AQ72L was specifically immunoprecipitated, but not GFP-Rab6AT27N (GDP-locked mutant). GFP-Rab6Awt was only poorly recovered, which suggests either that the steady state proportion of Rab6•GTP is indeed low in cells, or that the GTP-conformation was not properly retained after cell lysis. In either case, this may explain why no Rab6 effector has been efficiently co-immunoprecipitated with endogenous Rab6 under these conditions.

### f) detection of Rab6•GTP by immunofluorescence

Specific detection of Rab6•GTP by AA2 was also observed by immunofluorescence. HeLa cells expressing GFP-Rab6Q72L or GFP-Rab6T27N were co-stained with AA2 and an anti-Rab6 polyclonal. In GFP-Rab6Q72L expressing cells (Figure 3 A, B, outlined cells), both AA2 and the anti-Rab6 polyclonal showed an increased staining of the Golgi and of tubular and punctuate cytoplasmic structures. In cells expressing GFP-Rab6AT27N (Figure 3 C-F, outlined cells), while the polyclonal antibody detected the overexpressed GDP-locked Rab6 mutant in the cytosol, AA2 did not. In these cells, AA2 still detected endogenous Rab6 that remained localized at the Golgi. Thus AA2 detected Rab6•GTP, but not Rab6•GDP by immunofluorescence. This allowed the observation of the behavior of endogenous Rab6 upon overexpression of the GDP-locked mutant.

### g) Observation of the behaviour of endogeneous Rab6

Endogenous Rab6•GTP localized to Golgi membranes was not efficiently displaced upon overexpression of Rab6AT27N (Figure 3 E, F). In contrast, a total displacement of endogenous Rab6 was often observed upon overexpression of Rab6A'T27N (Figure 3 G, H). The use , according to the invention of a conformation-specific antibody thus allowed to draw two conclusions:
. firstly, because Rab6A'T27N perturbed both Rab6A and Rab6A', displacing them from the Golgi, it should not be used to determine the specific function of Rab6A' (***18***).
. secondly, because Rab6AT27N seemed less efficient in displacing Rab6 from Golgi membranes than Rab6A'T27N, specific partners of Rab6A'•GDP may exist (such as a specific exchange factor) that could not be titrated by overexpressed Rab6AT27N.

### h) Use of AA2 as a fluorescent intrabody

AA2 was used as a fluorescent intrabody to investigate Rab6•GTP dynamics *in vivo.* As Recombinant antibodies can be GFP-tagged and expressed in the cytoplasm of cultured cells in order to follow the behavior of endogenous proteins in living cells, without tagging and overexpressing the target protein **(*11*, *19*).** AA2 was fused to EYFP and expressed in HeLa cells. AA2-YFP co-localizes with Golgi markers in fixed cells (Figure S2) and living cells (Figure 4B), allowing the endogenous Rab6•GTP t obe *followed in vivo.* No effect on Rab6 function in intracellular traffic was observed upon AA2-YFP overexpression (Figure S2). Consistent with this, AA2 co-precipitated Rab6 and some of its effectors (Rab6IP2, BICD1, and p150glued, 16, 20, 21) in HeLa cells detergent extracts (Figure S2), suggesting that AA2 binding to Rab6•GTP did not necessarily inhibit the recruitment of its effectors.

### i) Use of AA2-YFP to monitor the dynamics of Rab6•GTP in live HeLa cells (Figure 4A, Movie S1).

AA2-YFP stained the Golgi complex and short tubulo-vesicular structures emanating from the Golgi, moving towards the cell periphery and sometimes back to the Golgi (Figure 4A, arrows and arrowhead respectively). They converge into regions (Figure 4A, brackets) that likely represent ER entry sites (22). Thus activated Rab6 was present on the Golgi and on transport intermediates as well. The lifetime of Rab6•GTP molecules on Golgi and transported membranes, limited by GTP hydrolysis, was thus long enough to allow recruitment of AA2-YFP (and likely intracellular Rab6 effectors) onto these membranes.

The size and shape of structures stained by AA2-YFP were in perfect accordance with those observed after localization of endogenous Rab6 by immunofluorescence (see Figure 1A d-f). However, they were relatively small compared to the 5-20 µm-long tubular transport intermediates observed in cells expressing GFP-Rab6 (22). Three reasons may explain such a difference. (i) AA2 may perturb the formation of tubules. (ii) Rab6 may be concentrated in a GTP-bound conformation at the tip of tubules. (iii) These relatively small structures represent endogenous transport intermediates, and overexpression, even slight, of Rab6 may cause an extension and stabilization of tubular structures.

### j) Monitoring of the behavior of CFP-Rab6 when co-expressed with AA2-YFP (Figure 4B, Movie S2).

A complete co-localization of the two fluorescent signals was observed (Movie S2). Importantly, the dynamics of AA2-YFP in these co-expression conditions matched those already reported for GFP-Rab6A alone (22). Thus AA2 was not inhibiting the formation of long tubules and Rab6 was present in its GTP-bound form all along their length, and even slight overexpression of Rab6 was enough to increase their length and stabilize them. Thus the geometry of transport intermediates may be modulated by Rab6 activity and up-regulation of Rab6 may stabilize membrane tubules. This is in agreement with models suggesting that tubulation of membrane is related to the traffic rate.

### References and Notes

1. Y. Takai, T. Sasaki, T. Matozaki, *Physiol Rev* **81,** 153 (2001).
2. V. S. Kraynov *et al., Science* **290,** 333 (2000).
3. Z. Li, C. D. Aizenman, H. T. Cline, *Neuron* **33,** 741 (2002).
4. S. H. Kim, Z. Li, D. B. Sacks, *J. Biol. Chem.* **275,** 36999 (2000).
5. P. Kalab, K. Weis, R. Heald, *Science* **295,** 2452 (2002).
6. K. Plafker, I. G. Macara, *J. Biol. Chem.* **277,** 30121 (2002).
7. G. Winter, A. D. Griffiths, R. E. Hawkins, H. R. Hoogenboom, *Annu. Rev. Immunol.* **12**, 433 (1994).
8. J. F. Smothers, S. Henikoff, P. Carter, *Science* **298,** 621 (2002).
9. S. Biocca, A. Cattaneo, *Trends Cell Biol.* **5,** 59 (1995).
10. P. A. Cohen, *Methods Mol Biol* **178,** 367 (2002).

11. F. Darchen, B. Goud, *Biochimie* **82,** 375 (2000).
12. *Information on materials and methods is available on Science Online.*
13. A. Echard *et al., Mol Biol Cell* **11,** 3819 (2000).
14. N. Nishimura, H. Nakamura, Y. Takai, K. Sano, *J Biol Chem* **269,** 14191 (1994).
15. S. Monier, F. Jollivet, I. Janoueix-Lerosey, L. Johannes, B. Goud, *Traffic* **3,** 289 (2002).
16. B. Stahl, G. Fischer von Mollard, C. Walch-Solimena, R. Jahn, *J. Biol. Chem.* **269,** 24770 (1994).
17. F. Mallard *et al., J Cell Biol* **156,** 653 (2002).
18. C. Nizak *et al., submitted for publication.*
19. T. Matanis *et al., Nat Cell Biol* ***4,*** 986 (2002).
20. B. Short, C. Preisinger, J. Schaletzky, R. Kopajtich, F. A. Barr, *Curr Biol* ***12,*** 1792 (2002).
21. J. White *et al., J Cell Biol* **147,** 743 (1999).
22. A. A. der Maur *et al., J Biol Chem* **277,** 45075 (2002).

S1. S. Monier, F. Jollivet, I. Janoueix-Lerosey, L. Johannes, B. Goud, *Traffic* 3, 289 (2002).
S2. T. Rowe, W. E. Balch, *Methods Enzymol* 257, 49 (1995).
S3. C. Nuoffer, F. Peter, W. E. Balch, *Methods Enzymol* 257, 3 (1995).
S4. O. Martinez *et al*., *Proc Natl Acad Sci U S A* 94, 1828 (1997).
S5. F. Mallard *et al*., *J. Cell Biol.* 143, 973 (1998).
S6. M. Jordan, A. Schallhorn, F. M. Wurm, *Nucleic Acids Res* 24, 596 (1996).
S7. S. M. *Elbashir et al., Nature* 411, 494 (2001).
S8. T. Matanis *et al., Nat Cell Biol 4,* 986 (2002).
S9. F. Mallard *et al*., *J Cell Biol* 156, 653 (2002).
S10. A. Girod *et al., Nat Cell Biol* 1, 423 (1999).
S11. J. White *et al., J Cell Biol 147,* 743 (1999).

## Claims

1. Use of scFv fragments as conformation-specific antibodies for specifically detecting a conformational protein state.

2. The use according to claim 1, wherein said scFv fragments are selected from a combinatorial library of scFv presented at the surface of phages.

3. The use according to claim 1 or 2, in immunofluorescence to determine the subcellular localization of the protein in a specific conformation.

4. The use according to claim 1 or 2, for detecting the expression of mutated proteins locked in a particular conformation.

5. The use according to claim 4, for detecting the presence of mutated proteins involved in pathologies.

6. The use of said scFv fragments for detecting by immunoprecipitation the presence of proteins in particular conformation.

7. The use according to cliam 6, for detecting the presence of mutated proteins in conditions where no overexpression at the nucleic acid level would be visible.

8. The use of said scFv according to claim 1 or 2,
